(19) Europäisches Patentamt
European Patent Office
Office européen des brevets

(11) Publication number: **0 097 516**
**B1**

(12) # EUROPEAN PATENT SPECIFICATION

(45) Date of publication of patent specification: **23.08.89**

(21) Application number: **83303540.5**

(22) Date of filing: **20.06.83**

(51) Int. Cl.[4]: **C 08 F 2/44,** C 08 F 230/08, C 12 N 11/08, C 12 N 5/00, G 01 N 33/53

(54) **Filler-containing polymer particles useful as carriers for supporting a biological substance.**

(30) Priority: **18.06.82 JP 103998/82**
**29.06.82 JP 110865/82**
**30.07.82 JP 132081/82**

(43) Date of publication of application:
**04.01.84 Bulletin 84/01**

(45) Publication of the grant of the patent:
**23.08.89 Bulletin 89/34**

(84) Designated Contracting States:
**DE FR GB**

(56) References cited:
**EP-A-0 011 186**
**EP-A-0 011 190**
**US-A-3 324 074**

(73) Proprietor: **JAPAN SYNTHETIC RUBBER CO., LTD.**
**11-24, Tsukiji-2-chome Chuo-ku**
**Tokyo 104 (JP)**

(72) Inventor: **Tai, Seiji**
**JSR Shataku 4-201 29, Aobadai-2-chome**
**Midori-ku Yokohama (JP)**
Inventor: **Kanayama, Hisanori**
**23-8, Ogawa-2-chome**
**Machida-shi (JP)**

(74) Representative: **Tubby, David George et al**
**MARKS & CLERK 57-60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 097 516 B1**

## Description

This invention relates to the use of a filler-containing polymer particles of an ethylenically unsaturated compound, which filler-containing polymer particles are useful as carriers for supporting a biological substance such as an immunoreactive substance, an enzyme, a microorganism and an organism cell.

Filler-containing polymer particles have conventionally been produced by mixing a filler with a polymer produced by a conventional polymerization method. There are, for example, a process which comprises mixing a filler with a solution of a polymer in an organic solvent, sufficiently stirring the solution to obtain a homogeneous mixture, and then spraying and drying the mixture; a process which comprises mechanically dispersing in water a filler-containing polymer solution, and then recovering an organic solvent; and a process which comprises kneading a polymer together with a filler, and then grinding the resulting mixture. These processes are disadvantageous, for example, in that a means for removing a solvent is needed, and in that the condition of dispersion of a filler in polymer particles is not uniform, so that the shapes and diameters of the polymer particles are irregular.

In order to remove these disadvantages, there has been proposed a process which comprises adding a filler to a monomer, and suspension-polymerizing the monomer to prepare a polymer. However, in the case of heretofore proposed suspension polymerization methods, the kinds of uniformly dispersible filler are limited, and the utilization of the resulting particles in which the shapes of the particles are retained is not taken into consideration, so that the strength of the particles is low. In the case of the conventional methods, the kind and amount of monomer are limited in conducting suspension polymerization. Further, the properties of a filler contained in the polymer particles are utilized, and when the surfaces of the polymer particles are required to be composed of a polymer alone, for example, when the polymer particles are used as a carrier for absorbing thereon or combining therewith a biological substance such as an immunoreactive substance, an enzyme, a microorganism, or an organism cell conventional filler-containing polymer particles are unsuitable for non-specifically supporting on their surface a biological substance because sometimes the filler is exposed on the surface.

This invention describes the provision of spherical filler-containing polymer particles which contain a filler in good dispersion condition, have a high particle strength, and are only slightly swollen with even a good solvent.

Such polymer particles have a high particle strength and contain in their inner parts a filler in a good dispersion condition and contain no filler in their surfaces.

This invention consists in the use as a carrier for supporting a biological substance of a filler-containing polymer particle, which comprises a polymer particle of an ethylenically unsaturated compound in which either 15% by weight of more of a polyfunctional ethylenically unsaturated compound or a silicon compound having an alkenyl group (hereinafter referred to as the alkenyl-silane compound) has been polymerized or copolymerized, and a filler dispersed in said polymer particle (said polymer particle is hereinafter referred to as the polymer particle (I).

According to this invention, there is also used a polymer particle (hereinafter referred to as the polymer particle (II) consisting of the polymer particle (I) and a filler-free polymer or copolymer of an ethylenically unsaturated compound formed on the surface of the polymer particle (I).

The polyfunctional ethylenically unsaturated compound which is a monomer used in this invention is, for example, a compound having in the molecule two or more α,β ethylenic unsaturations, and includes for instance, divinylbenzene; vinyl acrylate; vinyl methacrylate; polyester or acrylic acid or methacrylic acid with a polyol such as ethylene glycol, polyoxyethylene, propylene glycol, polyoxypropylene, trimethylolpropane, or glycerin; polyethers of allyl alcohol with said polyols. These polyfunctional ethylenically unsaturated compounds may be used alone or in combination of two or more. The amount of these polyfunctional ethylenically unsaturated compounds polymerized or copolymerized is 15 to 100% by weight, preferably 20 to 100% by weight. When it is less than 15% by weight, the resulting polymer particle is insufficient in strength and sometimes destroyed when supersonic treatment is carried out in using them; the polymer particle is high in the degree of swelling with a good solvent; and the filler contained in the polymer particles is localized therein, so that the color tones of the polymer particles become uneven.

The alkenyl-silane compound which is a monomer used in this invention can be represented, for example, by the following general formula:

$$X_nSiR_{4-n}$$

wherein X is a halogen atom, $—OR^1$ or

$$—O\overset{\overset{O}{\|}}{C}—R^1;$$

R is

$-CH=CH_2$, $-R^2O\overset{\overset{O}{\|}}{C}CH=CH_2$, $-R^2O\overset{\overset{O}{\|}}{C}-\overset{\overset{CH_3}{|}}{C}=CH_2$ or 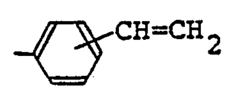 ;

n is 1, 2 or 3; $R^1$ is an alkyl group; and $R^2$ is a divalent saturated aliphatic group.

From the viewpoint of the toughness of or the polymerizability for a polymer or copolymer of the alkenyl-silane compound, X is preferably a halogen atom or $—OR^1$ and as the halogen atom, a chlorine atom is particularly preferred, while $R^1$ is preferably $—CH_3$ or $—C_2H_5$. As R, $—CH=CH_2$,

$$—R^2O\overset{O}{\overset{\|}{C}}CH=CH_2 \quad \text{and} \quad —R^2O\overset{O}{\overset{\|}{C}}\overset{CH_3}{\overset{|}{C}}=CH_2$$

are preferred, and $R^2$ is preferably $—C_2H_4—$ or $—C_3H_6—$.

The alkenyl-silane compound represented by the above general formula includes, for example, vinyltrichlorosilane, divinyldimethoxysilane, vinyltrimethoxysilane, vinyltriethoxysilane, di-γ-methacryloxypropyldimethoxysilane, γ-methacryloxypropyltrimethoxysilane, styryltrimethoxysilane, styryltriethoxysilane, etc.

These alkenyl-silane compounds may be used alone or even in combination of two or more.

The amount of these alkenyl-silane compounds polymerized or copolymerized is preferably 5 to 100% by weight, particularly preferably 5 to 50% by weight.

When it is less than 5% by weight, the dispersibility of the filler is insufficient and the particle strength is low. Moreover, the kinds and amounts of copolymerizable monomers are limited, and when a highly hydrophilic monomer is added, the filler is localized and, in the worst case, moves into the aqueous phase.

Other ethylenically unsaturated compounds copolymerizable with the polyfunctional ethylenically unsaturated compound or the alkenyl-silane compound are aromatic alkenyl compounds, unsaturated carboxylic acid compounds, unsaturated ester compounds, unsaturated amide compounds, unsaturated nitrile compounds, halogenated vinyl compounds and alkenyl lower aliphatic acid ester compounds, such as styrene, α-methylstyrene, vinyltoluene, ethylstyrene, chlorostyrene, sodium styrenesulfonate, methyl acrylate, methyl methacrylate, ethyl acrylate, ethyl methacrylate, n-butyl acrylate, n-butyl methacrylate, 2-ethylhexyl acrylate, 2-ethylhexyl methacrylate, glycidyl acrylate, glycidyl methacrylate, 2-hydroxyethyl acrylate, 2-hydroxyethyl methacrylate, polyoxyethylene acrylate, polyoxyethylene methacrylate, polyoxypropylene acrylate, polyoxypropylene methacrylate, 2-dimethylaminoethyl acrylate, 2-diethylaminoethyl acrylate, 2-dimethylaminoethyl methacrylate, 2-diethylaminoethyl methacrylate, acrylonitrile, methacrylonitrile, acrolein, methacrolein, acrylamide, methacrylamide, N-methylolacrylamide, methylenebisacrylamide, vinyl chloride, vinyl bromide, vinylidene chloride, vinyl acetate, N-vinylpyrrolidone, vinylpyridine, acrylic acid, methacrylic acid, itaconic acid, maleic acid and fumaric acid.

Water-soluble ethylenically unsaturated compounds such as acrylic acid, methacrylic acid, itaconic acid, acrylamide and methacrylamide are preferably used in an amount of 20% by weight or less.

A conjugated diene compound such as butadiene, isoprene or piperylene or the like may be copolymerized in such an extent that the polymer particles (I) do not adhere to one another to aggregate.

Such monomers to be copolymerized with the polyfunctional ethylenically unsaturated compound or the alkenyl-silane compound may be used alone or even in combination of two or more. Needless to say, the polyfunctional ethylenically unsaturated compound may be copolymerized with the alkenyl-silane compound, and in this case, it is sufficient that either the polyfunctional ethylenically unsaturated compound or the alkenyl-silane compound is copolymerized in a required amount. The simultaneous use of the polyfunctional ethylenically unsaturated compound and the alkenyl-silane compound further improves the dispersibility of the filler, and the strength, swelling-resistance and the like of the particles, etc.

The filler used in this invention includes, for example, metals such as iron, cobalt, nickel, copper, or aluminium; alloys of these metals with one another or with other metals, e.g. lanthanum series elements such as lanthanum, or gadolinium; metal oxides such as iron oxide, cobalt oxide, lead oxide, aluminium oxide, zinc oxide, silicon oxide, or titanium oxide; metal salts such as magnesium carbonate, calcium carbonate, aluminum silicate, barium sulfate, lead carbonate, lead chromate, cobalt aluminate, or mercurous chloride; metal sulfides such as zinc sulfide, or cadmium sulfide; and pigments or metallic chelate compounds such as Nickel Metallized Azo Yellow, Red Lake R, Permanent Red 2B, Copper Phthalocyanine Blue, or Copper Phthalocyanine Green. The particle diameter of the filler should be smaller than that of the desired polymer particle (I), and is desirably 30 Å to 10 μm, in particular, about 100 Å to 1 μm.

The surfaces of these fillers are often hydrophobic, and when the fillers are used as they are, the filler particles often aggregate in an oleophilic monomer and are, in some cases, difficult to disperse uniformly in the polymer particles. In this invention, particularly when no alkenyl-silane compound is used, there is suitably used a filler treated so as to impart an oleophilic property to the surface of the filler in order that the filler may uniformly be dispersed in an oleophilic monomer and that the filler particles do not aggregate and precipitate even when allowed to stand for a long time.

As the treatment method for imparting an oleophilic property to the surface of filler, there may be used, for example, a method for imparting an oleophilic property to the surface of filler which comprises contacting a filler with a surfactant composed mainly of a fatty acid in a medium such as water to form an adsorbed layer of the surfactant composed mainly of a fatty acid on the surface of the filler, followed by

washing the filler with an acidic solution having a pH value of less than 7, preferably a pH value of not less than 5 and less than 7 (Japanese Patent Application Kokai (Laid-Open) No. 22,688/76). The surfactant composed mainly of a fatty acid used for this purpose includes alkali metal salts of unsaturated fatty acids such as rosin acid, dodecenic acid, tetradecenic acid, hexadecenic acid and the like; alkali metal salts of saturated fatty acids such as myristic acid, palmitic acid, stearic acid, or arachic acid or alkaline earth metal salts of rosin acid.

As the acidic solutions having a pH value of less than 7, there may be used solutions prepared by adjusting the pH of a lower alcohol such as methanol or ethanol; a lower ketone such as acetone or methyl ethyl ketone or water with a mineral acid such as hydrochloric acid, sulfuric acid, nitric acid, or phosphoric acid.

The treatment temperature for forming an adsorbed layer of a surfactant composed mainly of a fatty acid on the surface of filler is usually 30°—150°C, preferably 70°—110°C. The treatment time is usually 0.5—2 hours, preferably 0.5—1 hour. Though the concentration of a surfactant composed mainly of fatty acid in the dispersion medium is not critical, it is usually 0.2% by weight or more, preferably 1—20% by weight.

It is considered that the filler treated by this method has formed thereon a fatty acid layer, where the hydrophilic group of the fatty acid is adsorbed on the filler surface and its oleophilic group faces outside, so that an oleophilic property is imparted to the filler surface.

As another treatment method for imparting an oleophilic property to the filler, there is a method which comprises contacting a filler with a compound having a very high filler-affinity portion and an oleophilic portion in its molecule, bonding the high filler-affinity portion to the filler by adsorption or chemical combination and treating the filler so as to direct the oleophilic portion to outside. As such a compound, there may be used silane coupling agents represented by the formula (1):

$$R^3_{4-m}SiR^4_m \qquad (1)$$

wherein $R^3$ is an alkoxy group preferably having 1—3 carbon atoms; an acyloxy group preferably having 2—4 carbon atoms, or a halogen atom; $R^4$ is a substituted or unsubstituted alkyl, alkenyl or aryl group, which includes, for example,

$-(CH_2)_3NH_2$, $-(CH_2)_3SH$, $-(CH_2)_3-NH-C_2H_5$, $-(CH_2)_3NH-(CH_2)_2NH_2$, $-CH=CH_2$, $-(CH_2)_3O-\underset{\underset{O}{\|}}{C}-C(CH_3)=CH_2$, $-(CH_2)_3O-CH_2-$

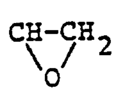 , $-(CH_2)_3Cl$, 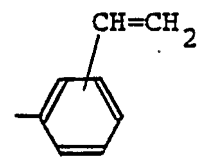

and the like; and m is 1, 2 or 3; titanate coupling agents represented by the formula (2):

$$R^5_pTiR^6_{4-p} \qquad (2)$$

wherein $R^5$ is a substituted or unsubstituted alkoxy, alkylenedioxy or acyloxy group, said alkoxy having preferably 1—6 carbon atoms, said acyloxy having preferably 2—12 carbon atoms, particularly preferably 2—4 carbon atoms, which includes, for example,

$CH_3O-$, $C_2H_5O-$, $C_3H_7O-$, $C_4H_9O-$, $CH_3C(CH_3)HO-$, 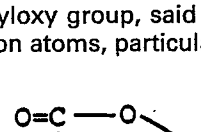 ,

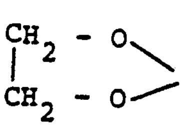 , 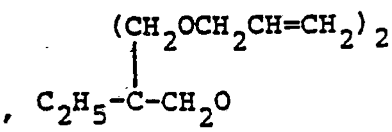

$R^6$ is an alkylacyloxy group having preferably 2—20 carbon atoms, a substituted benzene-sulfoxy group, a substituted phenoxy group, an unsaturated acyloxy group having preferably 2—6 carbon atoms, an alkyl

phosphate group or an alkyl phosphite group, which include, for example,

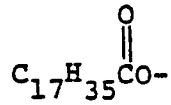 , 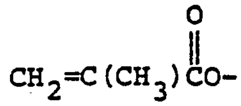 , 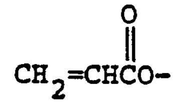 ,

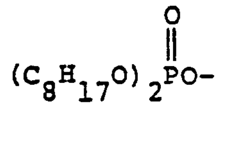 , 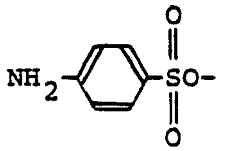 , 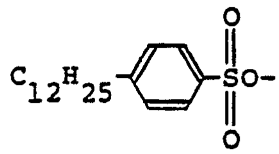 ,

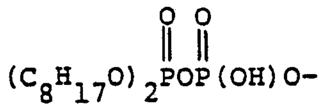 , 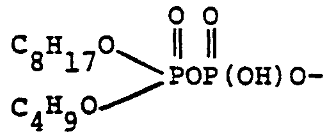 ,

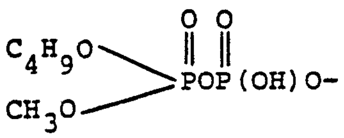 , $NH_2C_2H_4NHC_2H_4O-$ ,

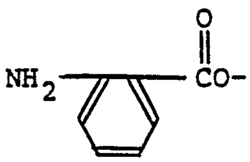 , 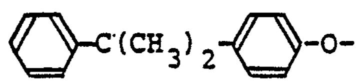 and p is 1, 2 or 3.

As the method for bonding a silane coupling agent or a titanate coupling agent to the filler, there may be used, for example, a method which comprises mixing the filler and the silane coupling agent or the titanate coupling agent in an organic medium such as water or an organic medium such as an alcohol; an ether, a ketone, or an ester, heating the resulting mixture with stirring, thereafter separating the filler by means of decantation and then removing the inorganic or organic medium by drying under reduced pressure. The silane coupling agent of the titanate coupling agent may be bonded to the filler by directly mixing the filler with the silane coupling agent or the titanate coupling agent without using any inorganic or organic medium and then heating the resulting mixture. In these methods, the heating temperature is usually 30°—100°C, and the heating time is about 30 minutes to 2 hours. Though the proportion of silane coupling agent or titanate coupling agent to filler is appropriately selected in consideration of the surface area of filler, it is usually 1—50 parts by weight, preferably 2—30 parts by weight, of the silane coupling agent or titanate coupling agent per 100 parts by weight of the filler. When an inorganic or organic medium is used, the concentration of the silane coupling agent or titanate coupling agent in the medium is usually 1% by weight or more, though it is not critical.

As other methods for imparting an oleophilic property to the filler, there may be used a method which comprises pulverizing the filler to finer particles by means of a pulverizing machine such as a ball mill in a hydrophobic medium containing a compound having a surface active effect such as a fatty acid, an alkyl sulfate, or an alkylarylsulfonic acid, thereby imparting an oleophilic property to the filler; a method which comprises adding and mixing an aqueous dispersion of fine powder of filler produced by a wet process or the like in the above-mentioned hydrophobic medium containing a surfactant to obtain an emulsion, thereafter heating the emulsion to distil off the water, thereby imparting an oleophilic property to the filler; and a method which comprises pre-heating the above-mentioned hydrophobic medium containing a surfactant, adding and mixing an aqueous dispersion of fine powder of filler produced by a wet process into the hydrophobic medium and simultaneously distilling off the water, thereby imparting an oleophilic property to the filler.

As a method for separating the filler having an oleophilic property obtained by these methods, from the hydrophobic medium, various methods may be used. For example, the filler can be precipitated by mixing the hydrophobic medium containing the filler having imparted thereto an oleophilic property with a lower alcohol miscible with the hydrophobic medium such as ethanol and propanol, and the precipitated filler can be collected by filtration or decantation.

The filler treated by these methods to give it an oleophilic property is very easily and stably dispersed in an oleophilic monomer and hardly causes solid-liquid separation. The filler is mixed with the monomer in an amount of preferably 0.1 to 200 parts by weight, particularly preferably 5 to 120 parts by weight, per 100 parts by weight of the monomer.

Processes for producing the polymer particle (I) of this invention are explained below.

Process A (Suspension polymerization method)

A monomer mixture is prepared by mixing the polyfunctional ethylenically unsaturated compound or the alkenyl silane compound and a polymerization initiator, if necessary, together with one or more other ethylenically unsaturated compounds to be copolymerized therewith and a filler. The aforesaid monomer mixture is poured into an aqueous medium comprising water as the main constituent, a suspension stabilizer and, if necessary, a surfactant, and the resulting mixture is stirred to form dispersed droplets of the monomer mixture. Depending on the constituents of the monomer mixture, heating may be effected for facilitating the formation of the dispersed droplets, or an organic solvent such as benzene, toluene, chloroform, ethyl acetate may be added or alternatively, the polymer dissolved in the monomer may be used.

The aforesaid suspension stabilizer and surfactant are used for preventing the dispersed droplets formed in the aqueous medium from being unified, and it is possible to control, by properly selecting their kinds and concentrations, the size of the dispersed droplets which depends dominantly on the degree of the stirring. As the suspension stabilizer, water-soluble polymers such as polyvinyl alcohol, sodium polyacrylate, polyvinyl pyrrolidone, polyoxyethylene, carboxymethyl cellulose, gelatin, or soluble starchs are preferred, and the suspension stabilizer is usually used in a proportion of 0.05 to 50 parts by weight per 100 parts by weight of the monomer mixture. As the aforesaid surfactants, there may be used fatty acid soaps, rosin soap, sodium alkylbenzenesulfonates, polyoxyethylene-polyoxypropylene block copolymers, polyoxyethylene fatty acid esters, polyoxyethylene alkylpheny ethers, or sorbitan fatty acid esters, and the surfactant is usually used in a proportion of 1 part by weight or less per 100 parts by weight of the monomer mixture.

As the aforesaid polymerization initiator, there may be used an alkyl peracid ester such as t-butyl perbenzoate, or isopropyl peracetate, an acyloyl peroxide such as benzoyl peroxide, or lauroyl peroxide; an aralkyl hydroperoxide such as cumene hydroperoxide, or p-menthane hydroperoxide; a dialkyl peroxide such as di-t-butyl peroxide; or an azobisacylonitrile such as azobisisobutyronitrile, or azobiscyclohexane-carbonitrile. The polymerization initiator is usually used in a proportion of about 0.1 to 5 parts by weight per 100 parts by weight of the monomer. If necessary, a molecular weight regulator such as t-dodecylmercaptan or carbon tetrachloride may be added to the aforesaid monomer mixture.

The amount of the aqueous medium used is usually 100 to 2,000 parts by weight, preferably 300 to 1,500 parts by weight, per 100 parts by weight of the monomer mixture. The stirring rate for forming the dispersed droplets of the monomer mixture is usually about 300 to 20,000 r.p.m.

In the case of using other ethylenically unsaturated compound to be copolymerized with the polyfunctional ethylenically unsaturated compound or the alkenyl-silane compound, when as the other ethylenically unsaturated compound to be copolymerized, there is used another ethylenically unsaturated compound having a high solubility in water and a high distribution ratio to the medium consisting of water, this other ethylenically unsaturated compound is preferably used in an amount of 20% by weight based on the amount of the total monomers. When said other ethylenically unsaturated compound is used in an amount of more than 20% by weight, in some cases, the diameter of the resulting polymer particles (I) is decreased, the localization of the filler is caused, and, moreover, the filler is moved to the aqueous phase.

Under the conditions described above, the dispersed droplets of the monomer mixture are formed in the aqueous medium, polymerization is caused by heating the system while continuing the stirring, whereby the polymer particles (I) are produced. The polymerization temperature is preferably 60° to 100°C. Although the average diameter of the thus obtained polymer particles (I) is varied depending upon the above-mentioned various conditions, the shape of the reactor and the like, it is usually about 0.1 μm to 2 mm. The polymer particles (I) produced are collected by a means such as a centrifugation method, a filtration method or the like and washed. If necessary, it is possible to classify the polymer particles (I) by a means such as a sieving method, a sedimentation separation method, and take out only those having a diameter in the desired range.

Process B (Solution-precipitation polymerization method)

A monomer mixture is prepared in the same manner as in the Process A and added to a liquid medium in which the polyfunctional ethylenically unsaturated compound, the alkenyl-silane compound, the other ethylenically unsaturated compound or compounds to be copolymerized therewith, and the polymerization initiator can be dissolved but a polymer to be produced cannot be dissolved or alternatively the constituents of the monomer mixture are separately added to the aforesaid liquid medium. The thus obtained mixture is heated with stirring to conduct polymerization, and the resulting polymer or copolymer is deposited in the liquid medium, whereby the polymer particles (I) are produced.

The liquid medium used in the above process is selected from solvents such as lower alcohols, ethers, ketones, halogenated hydrocarbons, aliphatic hydrocarbons, for example, methanol, ethanol, methyl ethyl

ketone, ethyl acetate, n-hexane, cyclohexane, 1,2-dichloroethane. However, when the other ethylenycally unsaturated compound or compounds to be polymerized are soluble in water and the polymer produced is insoluble in water, for example, when 2-hydroxyethyl acrylate, 2-hydroxyethyl methacrylate, acrolein, methacrolein is copolymerized, water or a mixed solution of water and an organic solvent miscible therewith may also be used.

It is sufficient that the dissolution of the monomers in the liquid medium in the present process is conducted at the polymerization temperature, and in some cases, the ionic monomer such as sodium styrenesulfonate or the like is copolymerized without dissolving it in a solvent.

As described above, in the present process, it is not always necessary to form, as in the process A, dispersed droplets of the monomer mixture in the liquid medium, and therefore, the employment of a suspension stabilizer and a surfactant becomes unnecessary.

The aforesaid polymerization initiator is soluble in the liquid medium used and, for example, the same polymerization initiators as in the process A may be used. When, for instance, a mixed solution of water and methanol is used as the liquid medium, a water-soluble radical polymerization initiator such as hydrogen peroxide, a persulfate may be used as the polymerization initiator. The other polymerization conditions are the same as in the process A. In the present process, there are obtained for instance, polymer particles having an average diameter of 0.05 to 100 μm by varying the kind and amount of the liquid medium.

The polymer particle (I) obtained by the above-mentioned Processes A and B is a spherical particle containing the filler uniformly dispersed therein, and their shapes are suitable for carrier.

The polymer particle (II) is produced by forming a filler-free polymer on the surface of the polymer particle (I) obtained in the manner described above (the filler-free polymer is hereinafter referred to as polymer (III)). As the ethylenically unsaturated compound for forming the polymer (III) on the surface of the polymer particle (I), there may be used the same compounds as used for the aforesaid polymer particle (I), but the employment of a polyfunctional ethylenically unsaturated compound or an alkenyl-silane compound is not essential. When a water-soluble ethylenically unsaturated compound is copolymerized, the amount thereof is such that the resulting copolymer is not soluble in water, for example, 50% by weight or less, preferably 30% by weight or less.

The weight ratio of the polymer particle (I) to the polymer (III) is preferably 100/5—200, more preferably 100/5—150, and most preferably 100/20—100. When the polymer (III) is formed in a ratio of less than 5 parts by weight per 100 parts by weight of the polymer particle (I), the polymer (III) cannot always cover the whole surface of the polymer particle (I), and the surface of the polymer particle (I) is exposed in some cases. When the polymer (III) is formed in a ratio of more than 200 parts by weight per 100 parts by weight of the polymer particle (I), the shell layer of the polymer (III) becomes very thick, so that no sufficient effect can be exerted in utilization of the physical properties of the filler in the inner part. Although the diameter of the polymer particles (II) is not critical, it is preferably 0.1 to 400 μm, particularly preferably 0.1 to 300 μm.

The method of forming the polymer (III) on the surface of the polymer particle (I) is not critical, and the formation is carried out, for example, by the following suspension polymerization method or emulsion polymerization method.

(a) Suspension polymerization method

The polymer particles (I) are dispersed in an organic phase comprising an ethylenically unsaturated compound, a polymerization initiator and optionally a chain transfer agent, and the resulting dispersion is dispersed in an aqueous phase in the presence of a suspension stabilizer at a temperature lower than the polymerization-initiating temperature. After droplets of the organic phase having a desirable particle diameter are obtained by varying the concentration of the suspension stabilizer and the stirring rate, the temperature is elevated to conduct polymerization, whereby the polymer particles (II) are obtained. The polymerization initiator, the chain transfer agent, the suspension stabilizer, and the polymerization conditions are the same as described for the above polymer particles (I).

(b) Emulsion polymerization method

The polymer particles (I) are dispersed in an aqueous phase containing a polymerization initiator, and an ethylenically unsaturated compound for forming the polymer (III) is charged thereinto at one time or continuously and polymerized in an emulsion state. As the polymerization initiator in this case, there may be used radical polymerization initiators usually used for emulsion polymerization, for example, persulfates; peroxides such as hydrogen peroxide, or metal peroxides; acyloyl peroxides; aralkyl hydroperoxides; esters or peracids, azonitriles, or the so-called redox catalyst systems formed by reacting a peroxide with a reducing agent in the presence of an iron salt.

As the reducing agent, there may be used acid sodium sulfate, sodium thiosulfate, or sodium metabisulfite. In addition to them, there may also be used polymerization adjuvants usually used for emulsion polymerization, for example, molecular weight regulators such as t-dodecylmercaptan, or carbon tetrachloride and chelating agents such as sodium ethylenediaminetetraacetate.

Although an emulsifier is not always needed, it may be used in an amount of, usually, about 1 part by weight or less, preferably 0.1 part by weight or less, per 100 parts by weight of the total monomer. As the emulsifier, there may be used anionic surfactants such as sodium dodecylbenzenesulfonate, potassium

oleate and nonionic surfactants such as polyoxyethylene lauryl ether and polyoxyethylene nonylphenyl ether. These may be used alone or in admixture of two or more. Although the pH at the time of the polymerization is not critical, it ranges, in general, from 2 to 10. Although the polymerization temperature also is not critical so long as it is in the range in which usual emulsion polymerization is conducted, temperatures in the range of 30° to 100°C are suitable.

In the polymer particle (I) of this invention, either 15% by weight or more of a polyfunctional ethylenically unsaturated compound or an alkenyl-silane compound has been polymerized or copolymerized, and therefore, as compared with a conventional filler-containing polymer particle, said polymer particle (I) has greatly increased physical strengths and can have a low degree of swelling even when immersed in a good solvent. Accordingly, the polymer particle (I) of this invention is hardly broken up even by various treatments, e.g., supersonic treatment and is not dissolved or swollen even when immersed, for example, in a monomer having a functional group for the purpose of further modifying the surface of the polymer particle (I). Also, a different monomer can be polymerized on the surface of said polymer particle (I).

Further, owing to the employment of either an alkenyl-silane compound or 15% by weight or more of a polyfunctional ethylenically unsaturated compound, a hydrophilic monomer may be added and used without causing the localization or separation of the filler, and the polar group of the hydrophilic monomer can be utilized.

In particular, the polymer particle (I) is characterized in that it contains a filler in a good dispersion state and is perfectly spherical. The above-mentioned Processes A and B are production processes in which substantially no filler-free polymer particle is formed at the time of the polymerization.

Further, the polymer particles (I) are very useful because the physical properties of the filler contained in their interior can be utilized. For instance, when a filler having a very high density is used, the resulting polymer particles have a high density and can easily be separated from the liquid medium by means of gravity or centrifugal force. When a magnetic filler is used, it is made possible to move the resulting polymer particles in a liquid medium, a living body or a vessel by means of magnetic force. The polymer particle (I) of this invention having a small diameter has a very large surface area per unit volume and hence is suitable as a carrier in which the surface of the polymer particle is utilized.

As described above, the polymer particles (I) can be given various chemical or physical properties by properly varying the combination of the monomers with the filler, and hence are very useful.

In the production process of this invention, the kind of the monomer or monomers to be copolymerized may considerably freely be selected; for instance, the polymer particles (I) can be produced by using a highly hydrophilic monomer.

Further, the polymer particle (II) is characterized in that like the polymer particle (I), it is hardly broken up even by various treatments, e.g., supersonic treatment because the particle strength of the polymer particle (I) covered with the surface layer of the polymer (III) is very high.

In the polymer particle (II) the physical properties of the filler contained in the interior thereof can be utilized, and a filler-free polymer is formed on the surface of the particle. As the polymer (III) constituting the surface of the polymer particle (II), either a nonpolar polymer or a polar polymer having a functional group may freely be used, and the surface performance of the polymer particle can sufficiently be exerted. For example, when fine particles of a high-density substance are used as the filler, there are obtained polymer particles which have a high apparent density and whose surfaces are composed of a filler-free polymer alone. These polymer particles, like the polymer particles (I), can easily be separated from the surrounding liquid medium by utilizing gravity or centrifugal force, and hence, are very useful as a carrier for biological substances. When a magnetic filler is used as in the case of the polymer particles (I), it becomes possible to separate the resulting polymer particles or move them in a liquid medium, a living body or a vessel by means of a magnetic force. Thus, in the case of the polymer particle (II) the physical properties of the filler can be utilized, and moreover the surface of the polymer particle has the desired chemical properties. By properly selecting the diameter of the polymer particle, this polymer particle can be made particularly ideal for utilizing the surface of the polymer particle.

Further, when the surfaces of the polymer particle (I) and the polymer particle (II) are composed of a polymer or copolymer of an alkenyl-silane compound, said polymer particles (I) and (II) have an excellent supporting ability and hardly undergo non-specific aggregation even after a substance to be supported has been supported thereon.

The polymer particle (I) and the polymer particle (II) having the characteristics described above are useful as carriers for biological substances, for example, immunoreactive substances represented by antigens and antibodies such as surface antigen of B type hepatitis (HBs antigen), anti-HBs antibody, human chorionic gonadotropin (HCG antigen), anti-HCG antibody, immunoglobulin G, mycoplasma antigen, nucleic acid, nuclear protein, estrogen, anti-estrogen antibody and the like; enzymes such as glucose isomerase, glucose oxidase, α-amylase, papain and aminoacylase; and organism cells requiring a solid surface for their growth, such as fetal pneumocyte, renal cell, fibroblast and the like, and are particularly useful as carriers for the immunoreactive substances and the organism cells.

The present invention is further explained below in more detail referring to Examples.

## Example 1

To 1 liter of water containing 100 g of magnetite particles having an average particle diameter of 110Å prepared by a wet process was added 400 ml of a 20% by weight aqueous potassium oleate solution, and the resulting mixture was stirred at 90°C for 30 minutes. After being cooled, the pH of the mixture was adjusted to 6 with diluted hydrochloric acid.

Since the magnetite particles aggregated, they were filtered off, washed with two 300-ml portions of water at 80°C and then with two 300-ml portions of ethanol, and dried under reduced pressure. In a 1-liter, four-necked flask equipped with a stirrer, a reflux condenser, a dropping funnel and a thermometer was placed 400 ml of a 3% by weight aqueous polyvinyl alcohol solution. On the other hand, 50 g of styrene, 100 g of divinylbenzene, 3 g of benzoyl peroxide, 1.5 g of t-dodecylmercaptan and 15 g of the magnetite particles obtained above having an oleophilic property imparted by the treatment with potassium oleate, were stirred at 3,000 rpm with water-cooling to uniformly mix them. Subsequently, the resulting mixture was dropped into the aforesaid four-necked flask through the dropping funnel while the aqueous polyvinyl alcohol solution contained in the flask was stirred. After completion of the dropping, the stirring of the contents was further continued for 30 minutes, and when the particle diameter of the organic phase became uniformly 25 μm, the contents were heated to 85°C to be subjected to polymerization. After the polymerization for 12 hours, the unreacted monomers were recovered under reduced pressure, and the polymer particles were separated by centrifugation and washed with three 500-ml portions of warm water, to obtain 140 g of polymer particles containing magnetite particles. The thus obtained polymer particles were observed under a microscope of 300 magnifications to find that the particles had a perfectly spherical shape having a diameter of 25 ± 10 μm and that the whole thereof had a uniform color tone. These polymer particles were attracted by a magnet, were not broken up even by supersonic treatment, and showed a degree of volume-swelling of 1.3 times even when immersed in toluene at room temperature for 5 hours.

## Example 2

Polymerization was conducted in the same manner as in Example 1, except that the 50 g of styrene was replaced by a mixture of 20 g of styrene and 30 g of methyl methacrylate. By the polymerization, 140 g of polymer particles containing magnetite particles were obtained. The polymer particles had a perfectly spherical shape having a diameter of 20 ± 10 μm and the whole thereof had a uniform color tone. The polymer particles were attracted by a magnet, were not broken up even by supersonic treatment, and showed a degree of volume-swelling of 1.2 times even when immersed in toluene at room temperature for 5 hours.

## Example 3

A solution of 5 ml of

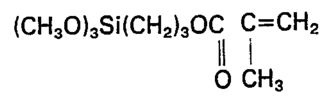

in 30 ml of ethanol was added at room temperature with stirring to 500 ml of ethanol which contained 15 g of ferrite particles having an average particle diameter of 80 μm prepared by a wet process. Subsequently, the resulting mixture was refluxed for 1 hour, cooled, centrifuged, then washed with two 20-ml portions of ethanol, and dried under reduced pressure. In a 1-liter flask was placed a homogeneous mixture prepared by previously stirring together, at 500 rpm 30 g of the ferrite particles having an oleophilic property imparted by the treatment described above, 10 g styrene, 90 g of divinylbenzene, 3 g of benzoyl peroxide, and 0.5 g of t-dodecylmercaptan, with ice-cooling. To the mixture was added 400 ml of a 1.5% aqueous polyvinyl alcohol solution with ice-cooling, after which the resulting mixture was stirred at 2,000 rpm for 15 minutes, and when the particle diameter of the suspended droplets became uniformly 12 μm, the mixture was heated to 80°C to be subjected to polymerization. After the polymerization was conducted for 16 hours, the unreacted monomers were recovered under reduced pressure, and the polymer particles were separated by centrifugation to obtain 100 g of polymer particles containing ferrite particles. The thus obtained polymer particles had a perfectly spherical shape having a diameter of 14 ± 5 μm, and the whole thereof had a uniform color tone. These polymer particles were not broken up even by supersonic treatment and showed a degree of volume-swelling of 1.1 times even when immersed in toluene at room temperature for 5 hours.

## Comparative Example 1

Polymerization was conducted in the same manner as in Example 1, except that the 50 g of styrene and the 100 g of the divinylbenzene were replaced by 135 g of styrene and 15 g of divinylbenzene, respectively. By the polymerization, 140 g of polymer particles containing magnetite particles were obtained. The polymer particles had a perfectly spherical shape having a diameter of 26 ± 8 μm, but the magnetite particles were localized as mass in the polymer particles. About 5% of the number of the polymer particles did not contain any filler. Further, a part of the polymer particles were broken up by supersonic treatment, and the degree of volume-swelling by immersion in toluene at room temperature for 1 hour was 10.6 times.

Example 4

| | |
|---|---|
| Fine particles of magnetite subjected to surface-treatment with potassium oleate in the same manner as in Example 1 | 30 g |
| n-Butyl methacrylate | 85 g |
| γ-Methacryloxypropyltrimethoxysilane | 15 g |
| Azobisisobutyronitrile | 3 g |

The above-mentioned materials were mixed to prepare a liquid monomer composition, which was then added to 300 ml of a 2% by weight aqueous polyvinyl alcohol solution and suspended therein by stirring. The resulting suspension was heated to a temperature of 80°C and subjected to polymerization for 8 hours while continuing the stirring at a rate of 600 rpm, whereby spherical, black polymer particles having an average diameter of 50 μm were produced.

The polymer particles contained the magnetite uniformly dispersed therein, were not easily broken up even by supersonic treatment, had magnetism due to the magnetite, and could be collected or moved by means of a magnet even in a dispersion. Further, the polymer particles had an excellent ability to support thermally denatured immunoglobulin G, and were suitable for carrier, and these particles could easily be separated and recovered by utilizing their sensitivity to a magnet.

The polymer particles can be improved in performance characteristics as a specific carrier by the improvement of their surface characteristics, for example, the introduction of a carboxyl group by the hydrolysis treatment of the particle surfaces.

Comparative Example 2

Polymerization was conducted in the same manner as in Example 4, except that the γ-methacryloxypropyltrimethoxysilane was omitted. The resulting polymer particles contained the magnetite dispersed therein, but they showed an uneven dispersion state of magnetite and were easily broken up by supersonic treatment.

Example 5

| | |
|---|---|
| Carbon black (particle diameter: 500—1,000Å | 10 g |
| Styrene | 70 g |
| Acrylonitrile | 20 g |
| Styryltrimethoxysilane | 10 g |
| Benzoyl peroxide | 3 g |

The above-mentioned materials were mixed to prepare a liquid monomer composition, which was then added to 300 ml of a 1% by weight aqueous polyvinyl alcohol solution and suspended therein by stirring. The resulting suspension was heated to 80°C and subjected to polymerization for 6 hours while continuing the stirring at a rate of 300 rpm, to produce spherical, black polymer particles having an average diameter of 0.4 mm.

The polymer particles contained the carbon black uniformly dispersed therein, and were difficult to break up even by supersonic treatment. Further, they had an excellent ability to support thermally denatured immunoglobulin G and were suitable for carrier.

Example 6

| | |
|---|---|
| Fine particles of magnetite not subjected to surface-treatment (particle diameter: 100—300Å) | 80 g |
| Styrene | 70 g |
| γ-Methacryloxypropyltrimethoxysilane | 30 g |
| Benzoyl peroxide | 5 g |

The above-mentioned materials were mixed to prepare a liquid monomer composition, which was then added to 500 ml of a 2.5% by weight aqueous polyvinyl alcohol solution and suspended therein by stirring. The resulting suspension was heated to 80°C and subjected to polymerization for 8 hours while continuing the stirring at a rate of 600 rpm, to produce spherical, black polymer particles having an average diameter of 15 μm.

The polymer particles contained the magnetite uniformly dispersed therein, were difficult to break up by supersonic treatment, had an excellent ability to support anti-HBs antibody and thermally denatured immunoglobulin G, and were useful for carrier.

Example 7

| | |
|---|---|
| Methyl methacrylate | 60 g |
| 2-Hydroxyethyl methacrylate | 10 g |
| Vinyltriethoxysilane | 30 g |
| Azobisisobutyronitrile | 1 g |

The above-mentioned materials were dissolved in 300 ml of ethyl alcohol, and into the resulting solution was dispersed 30 g of fine particles of ferrite (average length of the major axis: 300Å) which had not been subjected to the surface treatment, after which the resulting dispersion was heated to 70°C and subjected to polymerization for 6 hours while stirring it at a rate of 500 rpm, to produce spherical polymer particles having an average diameter of 0.2 μm.

The thus obtained polymer particles contained the ferrite uniformly dispersed therein, had a uniform sensitivity to a magnet, and were not easily broken up by supersonic treatment. The polymer particles had an excellent ability to support anti-HBs antibody and were useful as a carrier.

Example 8

In a mixed solution of 40 g of styrene, 10 g of γ-methacryloxytrimethoxysilane and 1 g of benzoyl peroxide was dispersed 50 g of the polymer particles obtained in Example 6, and the resulting dispersion was suspended in 300 ml of a 1% by weight aqueous polyvinyl alcohol solution. Subsequently, the resulting suspension was heated to 80°C and subjected to polymerization for 8 hours while continuously stirring it at a rate of 500 rpm, to produce spherical polymer particles having an average diameter of 100 μm.

This polymer particle contained a plurality of the polymer particles of Example 6, and the whole surface thereof was coated with a styrene-γ-methacryloxytrimethoxysilane copolymer.

Further, the polymer particles were not easily broken up even by supersonic treatment, had an excellent ability to support thermally denatured immunoglobulin G, and were suitable for carrier.

Example 9

In a 1-liter, four-necked flask equipped with a stirrer, a reflux condenser, a dropping funnel and a thermometer was placed 400 ml of a 3% by weight aqueous polyvinyl alcohol solution. To the solution was added a homogeneous mixture separately prepared by stirring at 600 rpm 80 g of styrene, 20 g of acrylonitrile, 2 g of benzoyl peroxide, 1.0 g of t-dodecylmercaptan and 100 g of the polymer particles obtained in Example 6 with ice-cooling. The resulting mixture was stirred at 3,500 rpm with ice-cooling for 30 minutes, and when the particle diameter of the organic phase reached 7 μm, the mixture was heated ot 85°C and subjected to polymerization for 5 hours. Thereafter, the unreacted monomers were recovered under reduced pressure, and the polymer particles were separated by centrifugation and then washed with three 500-ml portions of warm water. There were produced 185 g of polymer particles. As a result of observation under a microscope, the average particle size was found to be 4.4 μm. The elements present on the surfaces of the polymer particles obtained were examined by use of ESCA (electron spectroscopy for chemical analysis) in the usual manner. As a result, there were shown only absorptions due to carbon derived from the polymer and due to oxygen in the air adsorbed on the polymer surface, and the presence of iron element was not detected. The polymer particles were attracted by a magnet.

Example 10

With 100 g of the polymer particles obtained in Example 1 were mixed 30 g of styrene, 10 g of methacrylic acid, 2 g of benzoyl peroxide and 0.3 g of t-dodecylmercaptan, and the resulting mixture was subjected to polymerization in the same manner as in Example 1 to obtain polymer particles. The polymerization conversion was 80%. The surfaces of the polymer particles were subjected to measurement by use of ESCA as in Example 9. As a result, the presence of carbon and oxygen on the surfaces of the polymer particles was ascertained but iron was not detected thereon. The polymer particles were attracted by a magnet, and had an average diameter of 30.9 μm.

Example 11

A solution prepared by dissolving 20 ml of

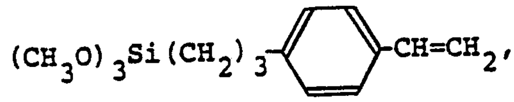

which was a silane coupling agent, in 200 ml of ethanol was added dropwise at room temperature with stirring to 1 liter of water containing 100 g of lead oxide particles having an average diameter of 200Å. The resulting mixture was heated and refluxed with stirring for 1 hour. The thus obtained mixed solution was cooled and then added to 3 liters of ethanol with stirring, after which the resulting precipitate was filtered off, washed with two 300-ml portions of ethanol, and then dried under reduced pressure. Suspension polymerization was conducted in the same manner as in Example 1, except that the 60 g of magnetite particles having an oleophilic property imparted by the treatment with potassium oleate in Example 1 was replaced by 60 g of the lead oxide particles obtained above having an oleophilic property imparted by the treatment with the silane coupling agent, to obtain 325 g of filler-containing polymer particles having an average diameter of 36 µm.

Further, 400 ml of a 2.5% by weight aqueous polyvinyl alcohol solution was placed in a 1-liter, four-necked flask equipped with a stirrer, a reflux condenser, a dropping funnel and a thermometer. On the other hand, 100 g of styrene, 20 g of 2-diethylaminoethyl methacrylate, 3.5 g of benzoyl peroxide, 0.5 of t-dodecylmercaptan, and 100 g of the polymer particles obtained above were previously stirred together at 3,500 rpm with ice-cooling for 30 minutes, and when the average particle diameter of the organic phase reached 70 µm, the thus obtained mixture was heated to 85°C and subjected to polymerization for 5 hours. The unreacted monomers were recovered under reduced pressure, and the polymer particles were separated by centrifugation and then washed with three 500-ml portions of warm water, to produce 205 g of polymer particles. The average particle diameter thereof was 47 µm as measured by means of a microscope. As a result of measurement using ESCA as in Example 9, the presence of carbon and oxygen on the surfaces of the polymer particles were ascertained, but no lead was detected thereon. The density of the polymer particles obtained was measured by use of a flotation-preceptation process in the usual way. The density was 1.28 g/ml.

Example 12

In 350 ml of water was dispersed 100 g of the polymer particles obtained in Example 1, after which 5 g of methacrylic acid and 95 g of styrene were added, and the resulting mixture was heated to 80°C with stirring. Subsequently, 0.5 g of potassium peroxide was added thereto, and the mixture thus obtained was subjected to polymerization at 80°C for 6 hours to obtain polymer particles. The polymerization conversion was 95%. The surfaces of the polymer particles were subjected to measurement by using ESCA as in Example 9. As a result, the presence of carbon and oxygen on the surfaces of the polymer particles was ascertained, but no iron was detected thereon. The polymer particles obtained were attracted by a magnet, and their average particle diameter was 45 µm.

Example 13

To 80 ml of MEM (a trade name for a culture medium of Nissui Seiyaku) containing 5% by weight of bovin embryoserum was added 0.5 g of the polymer particle obtained in Example 5. In the mixture was then suspended $4 \times 10^4$ V 79 cells (pneumal fibroblast of Chinese hamster). After allowing the suspension to stand for 4 hours in an atmosphere of air containing 5% by volume of carbon dioxide in order to attach the cells to the polymer particles, the cells were cultured for 7 days while slowly stirring the suspension with a teflon-made stirring rod. After completion of cultivation, a magnetic force was applied from outside of the culture vessel to collect the polymer particles to the bottom of the culture vessel, and then the vessel was inclined while maintaining the polymer particles in said state, whereby MEM was allowed to flow out. Then, the polymer particles were washed with phosphate buffer saline (−) solution, after which the polymer particles were collected to the bottom of the culture vessel and phosphate buffer saline (−) solution was allowed to flow out in the same manner as above. Then, 2 ml of 0.25% by weight phosphate buffer saline (−) solution of trypsin was added to peel off the cells from the surface of polymer particles, after which the polymer particles were collected to the bottom of vessel by magnetic force and the phosphate buffer saline (−) solution of trypsin was recovered in the same manner as above. The number of V 79 cells in this phosphate buffer saline (−) solution of trypsin was $3 \times 10^6$.

The result of this Example demonstrates the superiority of the polymer particle of this invention as a cell culture bed.

**Claims**

1. The use as a carrier for supporting a biological substance of a filler-containing polymer particle which comprises a particle of a polymer of an ethylenically unsaturated compound in which a silicon compound having an alkenyl group or at least 25% by weight of a polyfunctional ethylenically unsaturated compound is polymerized or copolymerized, and having a filler dispersed therein.

2. The use of a filler-containing polymer particle according to claim 1, wherein the silicon compound is at least one alkenyl-silane represented by the formula:

$$X_nSiR_{4-n}$$

wherein
X is a halogen atom, $—OR^1$ or

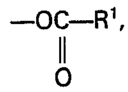

in which $R^1$ is an alkyl group;
R is

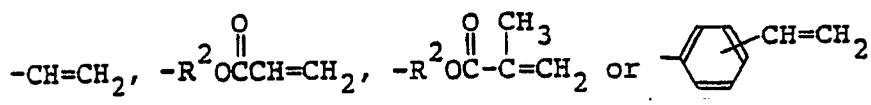

or ;

in which $R^2$ is a divalent saturated aliphatic group; and n is 1, 2 or 3.

3. The use of a filler-containing polymer particle according to claim 1 or claim 2, wherein the amount of said silicon compound polymerized or copolymerized is from 5 to 100% by weight.

4. The use of a filler-containing polymer particle according to any one of claims 1 to 3 , wherein the polyfunctional ethylenically unsaturated compound is at least one polyester of acrylic acid or methacrylic acid with a polyol; and/or polyether of allyl alcohol with a polyol; and/or divinylbenzene; and/or vinyl acrylate; and/or vinyl methacrylate.

5. The use of a filler-containing polymer particle according to claim 4, wherein the amount of said polyfunctional ethylenically unsaturated compound polymerized or copolymerized is 15 to 100% by weight.

6. The use of a filler-containing polymer particle according to any one of claims 1 to 5, wherein said silicon compound or said polyfunctional ethylenically unsaturated compound is copolymerized with another ethylenically unsaturated compound.

7. The use of a filler-containing polymer particle according to any one of the preceding claims, wherein the filler has a particle diameter of from 3 to $10^4$ nm.

8. The use of a filler-containing polymer particle which comprises (I) at least one filler-containing polymer particle as claimed in any one of claim 1 to 7 covered by (II) a filler-free layer of a polymer or copolymer of an ethylenically unsaturated compound or a silicon compound having an alkenyl group.

9. The use of a filler-containing polymer particle according to claim 8, wherein the filler-free layer of a polymer or copolymer is of the same or different monomer or monomers as the monomer or monomers constituting the polymer particle (I).

10. The use of a filler-containing polymer particle according to claim 8, wherein the weight ratio of the filler-containing polymer (I) to the polymer or copolymer (II) is from 100:5 to 100:200.

11. The use of a filler-containing polymer particle according to any one of claims 8 to 10, which has a particle diameter of from 0.1 to 400 µm.

**Patentansprüche**

1. Verwendung eines einen Füllstoff enthaltenden Polymerteilchens als Träger für eine biologische Substanz, gekennzeichnet durch ein Teilchen eines Polymers einer ethylenisch-ungsättigten Verbindung, in dem eine Siliciumverbindung mit einem Alkenylrest oder mindestens 15 Gew.-% einer polyfunktionellen ethylenisch-ungesättigten Verbindung polymerisiert oder copolymerisiert und ein Füllstoff dispergiert ist.

2. Verwendung eines einen Füllstoff enthaltenden Polymerteilchens nach Anspruch 1, in dem die Siliciumverbindung mindestens ein Alkenylsilan der Formel

$$X_nSiR_{4-n}$$

ist, in der bedeuten:
X ein Halogenatom, $—OR^1$ oder

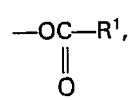

mit $R^1$ = Alkyl, R

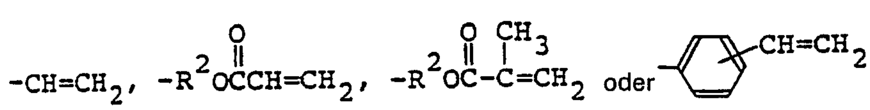

oder ;

mit $R^2$ = ein divalenter gesättigter aliphatischer Rest, und n 1, 2 oder 3.

3. Verwendung eines einen Füllstoff enthaltenden Polymerteilchens nach Anspruch 1 oder 2, in dem die Menge an polymerisierter oder copolymerisierter Siliciumverbindung von 5 bis 100 Gew.-% beträgt.

4. Verwendung eines einen Füllstoff enthaltenden Polymerteilchens nach einem der Ansprüche 1—3, in dem die polyfunktionelle ethylenische-ungesättigte Verbindung mindestens ein Acrylsäure- oder Methacrylsäure-Polyester mit einem Polyol und/oder ein Allylalkohol-Polyether mit einem Polyol und/oder Divinylbenzol und/oder Vinylacrylat und/oder Vinylmethacrylat ist.

5. Verwendung eines einen Füllstoff enthaltenden Polymerteilchens nach Anspruch 4, in dem die Menge an polymerisierter oder copolymerisierter, polyfunktioneller ethylenische-ungesättigter Verbindung von 15 bis 100 Gew.-% beträgt.

6. Verwendung eines einen Füllstoff enthaltenden Polymerteilchens nach einem der Ansprüche 1—5, in dem die Siliciumverbindung oder die polyfunktionelle ethylenisch-ungesättigte Verbindung mit einer anderen ethylenisch-ungesättigten Verbindung copolymerisiert ist.

7. Verwendung eines einen Füllstoff enthaltenden Polymerteilchens nach einem der vorstehenden Ansprüche, in dem der Füllstoff einen Teilchendurchmesser von 3 bis $10^4$ nm hat.

8. Verwendung eines einen Füllstoff enthaltenden Polymerteilchens, gekennzeichnet durch (I) mindestens ein einen Füllstoff enthaltendes Polymerteilchen nach einem der Ansprüche 1—7, das (II) mit einer füllstofffreien Schicht eines Polymers oder Copolymers einer ethylenisch-ungesättigten Verbindung oder einer Siliciumverbindung mit einem Alkenylrest überzogen ist.

9. Verwendung eines einen Füllstoff enthaltenden Polymerteilchens nach Anspruch 8, in dem die füllstofffreie Schicht eines Polymers oder Copolymers aus dem gleichen oder einem anderen Monomer oder Monomeren wie das Monomer oder die Monomere des Polymerteilchens (I) besteht.

10. Verwendung eines einen Füllstoff enthaltenden Polymerteilchens nach Anspruch 8, in dem das Gewichtsverhältnis des den Füllstoff enthaltenden Polymers (I) zu dem Polymer oder Copolymer (II) von 10:5 bis 100:200 beträgt.

11. Verwendung eines einen Füllstoff enthaltenden Polymerteilchens nach einem der Ansprüche 8—10, in dem das Teilchen einen Durchmesser von 0,1 bis 400 µm hat.

**Revendications**

1. Utilisation comme support d'une substance biologique, d'une particule de polymère contenant une charge, ladite particule comprenant une particule d'un polymère d'un composé à insaturation éthylénique dans lequel un composé à base de silicium ayant un groupe alkényle ou au moins 25% en poids d'un composé polyfonctionnel à insaturation éthylénique se trouve à l'état polymérisé ou copolymérisé, et une charge à l'état dispersé.

2. Utilisation d'une particule de polymère contenant une charge selon la revendication 1, dans laquelle le composé à base de silicium est au moins un alkényl-silane représenté par la formule

$$X_nSiR_{4-n}$$

dans laquelle

X est un atome d'halogène, $—OR^1$ ou

$$—O\underset{\underset{O}{\|}}{C}—R^1,$$

dans lesquelles $R^1$ est un groupe alkyle;

R est

$-CH=CH_2$, $-R^2O\overset{\overset{O}{\|}}{C}CH=CH_2$, $-R^2O\overset{\overset{O}{\|}}{C}-\overset{\overset{CH_3}{|}}{C}=CH_2$ ou 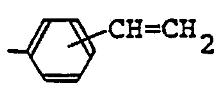 ;

dans lesquelles

$R^2$ est un groupe aliphatique divalent saturé; et n est 1, 2 ou 3.

3. Utilisation d'une particule de polymère contenant une charge selon la revendication 1 ou la revendication 2, dans laquelle la quantité dudit composé à base de silicium à l'état polymérisé ou copolymérisé est de 5 à 100% en poids.

4. Utilisation d'une particule de polymère contenant une charge selon l'une quelconque des revendications 1 à 3, dans laquelle le composé polyfonctionnel à insaturation éthylénique est au moins un polyester de l'acide acrylique ou méthacrylique avec un polyol; et/ou un polyéther d'alcool allylique avec un polyol; et/ou du divinylbenzène; et/ou de l'acrylate de vinyle; et/ou du méthacrylate de vinyle.

5. Utilisation d'une particule de polymère contenant une charge selon la revendication 4, dans laquelle la quantité dudit composé polyfonctionnel à insaturation éthylénique à l'état polymérisé ou copolymérisé est 15 à 100% en poids.

6. Utilisation d'une particule de polymère contenant une charge selon l'une quelconque des

revendications 1 à 5, dans laquelle ledit composé à base de silicium ou ledit composé polyfonctionnel à insaturation éthylénique est copolymérisé avec un autre composé à insaturation éthylénique.

7. Utilisation d'une particule de polymère contenant une charge selon l'une quelconque des revendications précédentes, dans laquelle la charge a un diamètre particulaire allant de 3 à $10^4$ nm.

8. Utilisation d'une particule de polymère contenant une charge comprenant (I) au moins une particule de polymère contenant (I) au moins une particule de polymère contenant une charge selon l'une quelconque des revendication 1 à 7 revêtue par (II) une couche exempte de charge d'un polymère ou copolymère d'un composé à insaturation éthylénique ou d'un composé à base de silicium ayant un groupe alkényle.

9. Utilisation d'une particule de polymère contenant une charge selon la revendication 8, dans laquelle la couche exempte de charge d'un polymère ou copolymère est formée du même ou des mêmes monomère(s) ou d'un ou de monomère(s) différent(s) du ou des monomère(s) constituant la particule de polymère (I).

10. Utilisation d'une particule de polymère contenant une charge selon la revendication 8, dans laquelle le rapport pondéral du polymère contenant une charge (I) au polymère ou copolymère (II) est de 100:5 à 100:200.

11. Utilisation d'une particule de polymère contenant une charge selon l'une quelconque des revendications 8 à 10, ayant un diamètre particulaire de 0,1 à 400 μm.